(19) Europäisches Patentamt
European Patent Office
Office européen des brevets

(11) Veröffentlichungsnummer: **0 040 179**
**B1**

(12) **EUROPÄISCHE PATENTSCHRIFT**

(45) Veröffentlichungstag der Patentschrift: **29.08.84**

(51) Int. Cl.³: **A 01 N 47/34**, C 07 D 213/64

(21) Anmeldenummer: **81810181.8**

(22) Anmeldetag: **08.05.81**

(54) **Phenylbenzoylharnstoffe.**

(30) Priorität: **14.05.80 CH 3777/80**

(43) Veröffentlichungstag der Anmeldung: **18.11.81 Patentblatt 81/46**

(45) Bekanntmachung des Hinweises auf die Patenterteilung: **29.08.84 Patentblatt 84/35**

(84) Benannte Vertragsstaaten: **AT BE CH DE FR GB IT LI NL**

(56) Entgegenhaltungen:
**EP - A - 0 025 363**
**DE - A - 2 818 830**
**US - A - 4 003 733**
**US - A - 4 005 223**
**US - A - 4 173 637**
**US - A - 4 173 638**

(73) Patentinhaber: **CIBA-GEIGY AG, Postfach, CH-4002 Basel (CH)**

(72) Erfinder: **Böger, Manfred, Wilhelm-Glock-Strasse 14, D-7858 Weil am Rhein 5 (DE)**
Erfinder: **Ehrenfreund, Josef, Dr., Langenhagweg 11, CH-4123 Allschwil (CH)**

**EP 0 040 179 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-3-(3-Chlor-5-trifluormethyl-2-pyridyloxy)-phenyl-N′-benzoylharnstoffe, Verfahren zu ihrer Herstellung und ihre Verwendung in der Schädlingsbekämpfung. Neue Ausgangsstoffe und deren Herstellung bilden ebenfalls einen Gegenstand der Erfindung.

Die erfindungsgemäßen halogensubstituierten N-3-(3-Chlor-5-trifluormethyl-2-pyridyloxy)-phenyl-N′—benzoylharnstoffe haben die Formel I

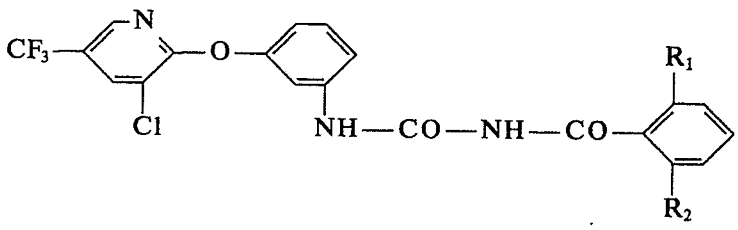
(I)

worin

$R_1$ Fluor, Chlor, Brom oder Methyl und
$R_2$ Wasserstoff, Fluor, Chlor oder Brom

bedeuten.

Wegen ihrer Wirkung als Schädlingsbekämpfungsmittel bevorzugt sind Verbindungen der Formel I, worin $R_1$ und $R_2$ Fluor oder Chlor bedeuten. Von besonderem Interesse sind ferner Verbindungen der Formel I, worin $R_2$ Wasserstoff bedeutet.

Die Verbindungen der Formel I können nach an sich bekannten Verfahren hergestellt werden (vgl. u. a. die deutschen Offenlegungsschriften Nr. 2 123 236, 2 601 780 und die japanische Patentschrift 5-3 103 447).

So kann man z. B. eine Verbindung der Formel I erhalten durch Umsetzung von

a) der Verbindung der Formel II

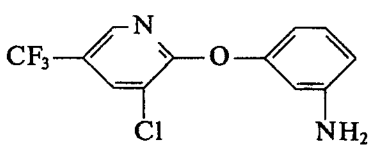
(II)

mit einer Verbindung der Formel III

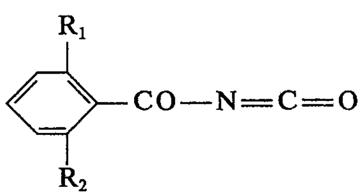
(III)

oder

b) der Verbindung der Formel IV

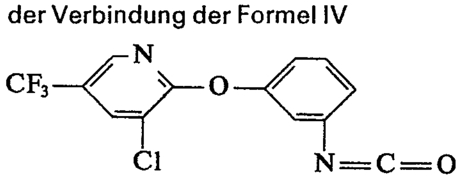
(IV)

gegebenenfalls in Gegenwart einer organischen oder anorganischen Base mit einer Verbindung der Formel V

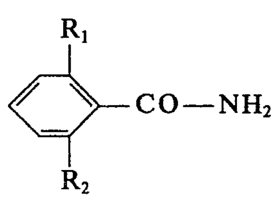
(V)

oder

c) der Verbindung der Formel II mit einer Verbindung der Formel VI

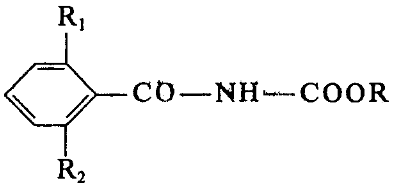

(VI)

In den obigen Formeln III, V und VI haben die Reste $R_1$ und $R_2$ die unter Formel I vorstehend angegebenen Bedeutungen und R bedeutet einen $C_1-C_8$-Alkylrest, der gegebenenfalls mit Halogen substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z. B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z. B. Aceton, Methyläthylketon, Methylisopropylketon und Methylisobutylketon. Verfahren a) wird im allgemeinen bei einer Temperatur von —10 bis 100°C, vorzugsweise zwischen 15 und 25°C, gegebenenfalls in Gegenwart einer organischen Base, z. B. Triäthylamin durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d. h. für die Umsetzung der Urethane der Formel VI mit dem Anilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylol, Chlorbenzol usw., verwendet werden.

Die Ausgangsstoffe der Formeln III und V sind bekannt und können analog bekannten Verfahren hergestellt werden. Bei den Ausgangsstoffen der Formeln II und IV handelt es sich um neuartige Verbindungen, die nach an sich bekannten Arbeitsweisen hergestellt werden können.

Das 3-(3-Chlor-5-trifluormethyl-pyridyl-2-oxy)-anilin der Formel II kann wie folgt erhalten werden:

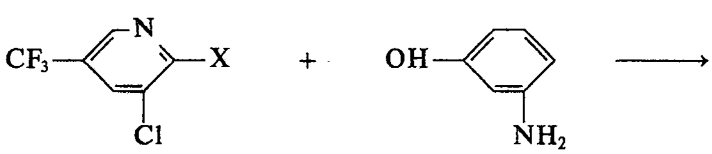

(II)

(X = Cl oder F)

Diese Umsetzung wird bei einer Temperatur von 20—180°C, vorzugsweise 50—160°C, in Gegenwart eines Säureacceptors, z. B. eines Alkali- oder Erdalkalihydroxids oder -hydrids, vorzugsweise KOH oder NaoH, sowie eines inerten organischen Lösungsmittels, vorzugsweise Dimethylformamid oder Dimethylsulfoxid, durchgeführt. Weiterhin ist das Anilin der Formel II in Analogie zu dem in J. Org. Chem. 29 (1964), 1, aufgezeigten Verfahren durch Hydrierung der entsprechenden Nitro-Verbindungen herstellbar (vgl. auch die dort zitierte Literatur). Auch durch chemische Reduktion (z. B. mittels Sn-(II)-Chlorid/HCl) der entsprechenden Nitroverbindung ist das Anilin der Formel II zugänglich (vgl. Houben Weyl, »Methoden d. org. Chemie« 11/1, 422).

Zu den Benzoylisocyanaten der Formel III kann man unter anderem wie folgt gelangen (vgl. J. Agr. Food Chem. 21, 348 und 993; 1973):

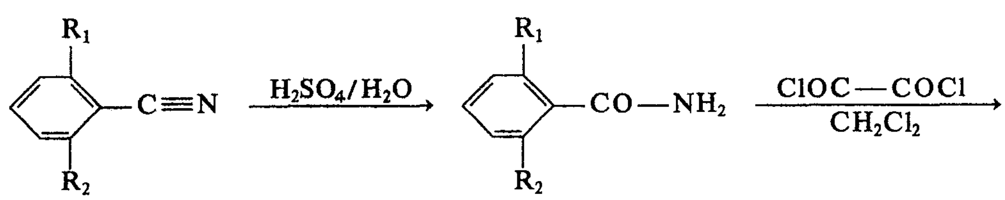


(III)

Das 3-(3-Chlor-5-trifluormethyl-pyridyl-2-oxy)-phenylisocyanat der Formel IV läßt sich z. B. durch Phosgenisierung des Anilins der Formel II nach allgemein üblichen Verfahren herstellen. Die weiterhin als Ausgangsstoffe zu verwendenden Benzamide der Formel V sind bekannt (vgl. z. B. Beilstein »Handbuch der organischen Chemie« Bd. 9, S. 336).

Die Urethane der Formel VI können in an sich bekannter Weise erhalten werden durch Umsetzung

eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Es ist bereits bekannt, daß bestimmte substituierte N-Phenoxyphenyl-N'-benzoylharnstoffe insektizide Eigenschaften besitzen. So sind aus den deutschen Offenlegungsschriften 2 504 982 und 2 537 413 halogensubstituierte N-4-(2-Chlor-4-trifluormethyl-phenoxy)-phenyl-N'-benzoylharnstoffe mit insektizider Wirkung bekannt. Auch die japanische Patentschrift 5-310 447 betrifft N-4-(Trifluormethylphenoxy)-phenyl-N'-benzoylharnstoffe als insektizide Wirkstoffe. Weiterhin werden in den deutschen Offenlegungsschriften 2 748 636 und 2 818 830 sowie in der japanischen Patentschrift 5-4 115 380 N-4-(2-Pyridyloxy)-phenyl-N'-benzoylharnstoffe mit insektizider Wirkung beschrieben.

Bei den erfindungsgemäßen Verbindungen der Formel I handelt es sich demgegenüber um neuartige substituierte N-3-(2-Pyridyloxy)-phenyl-N'-benzoylharnstoffe, die überraschenderweise erhöhte insektizide Wirksamkeit, insbesondere gegen fressende Schadinsekten, wie Spodoptera littoralis und Heliothis virescens, aufweisen. Nicht vorherzusehen war auch die außerordentlich hohe Wirksamkeit der Verbindungen der Formel I gegen Eier und Larven von Musca domestica und Aedes aegypti-Larven. Ein weiterer Vorteil der erfindungsgemäßen Verbindungen der Formel I ergibt sich aus ihrer sehr geringen Warmblütertoxizität bei guter Pflanzenverträglichkeit.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer Wirkung gegenüber Fliegen, wie z. B. Musca domestica, und Mückenlarven können Verbindungen der Formel I auch zur Bekämpfung von pflanzenschädigenden Fraßinsekten in Zier- und Nutzpflanzungen, insbesondere in Baumwollkulturen (z. B. gegen Spodoptera littoralis und Heliothis virescens), sowie in Obst- und Gemüsekulturen (z. B. gegen Laspeyresia pomonella, Leptiontarsa decemlineata und Epilachna varivestis) eingesetzt werden. Die Verbindungen der Formel I zeichnen sich durch eine ausgeprägte Wirkung gegen larvale Insektenstadien, insbesondere larvale Stadien fressender Schadinsekten, aus. Werden Verbindungen der Formel I von adulten Insekten-Stadien mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B., durch Tier-, Stall- und Weidebehandlung.

Die Wirkung der erfindungsgemäßen Verbindungen bzw. der sie enthaltenden Mittel läßt sich durch Zusatz von anderen Insektiziden und/oder Akariziden wesentlich verbreitern und an gegebene Umstände anpassen.

Als Zusätze kommen z. B. folgende Wirkstoffe in Betracht: organische Phosphorverbindungen, Nitrophenole und Derivate, Formamidine, Harnstoffe, Carbamate, chlorierte Kohlenwasserstoffe und bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u. a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Träger und/oder Zuschlagstoffen eingesetzt werden. Geeignete Träger und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind »cattle dips«, d. h. Viehbäder, und »spray races«, d. h. Sprühgänge, in denen wäßrige Zubereitungen verwendet werden, zu erwähnen. Diese Zubereitungsformen sind insbesondere zur Bekämpfung tierparasitärer Schädlinge geeignet.

Die Herstellung erfindungsgemäßer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate; Spritzpulver (wettable powders), Pasten, Emulsionen;
b) Lösungen.

Der Gehalt an Wirkstoff in den oben beschriebenen Mitteln liegt zwischen 0,1 bis 95%.

Die Wirkstoffe der Formel I können beispielswiese wie folgt formuliert werden:

### Stäubemittel

Zur Herstellung eines a) 5%igen und b) 2%igen Stäubemittels werden die folgenden Stoffe verwendet:

a) 5 Teile Wirkstoff,
   95 Teile Talkum;
b) 2 Teile Wirkstoff,
   1 Teil hochdisperse Kieselsäure,
   97 Teile Talkum.

Die Wirkstoffe werden mit den Trägerstoffen vermischt und vermahlen.

### Granulat

Zur Herstellung eines 5%igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngröße 0,3—0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschließend das Aceton im Vakuum verdampft.

### Spritzpulver

Zur Herstellung eines a) 40%igen, b) und c) 25%igen d) 10%igen Spritzpulvers werden folgende Bestandteile verwendet:

a) 40 Teile Wirkstoff,
   5 Teile Ligninsulfonsäure-Natriumsalz,
   1 Teil Dibutylnaphthalinsulfonsäure-Natriumsalz
   54 Teile Kieselsäure;

b) 25,0 Teile Wirkstoff,
   4,5 Teile Calcium-Ligninsulfonat,
   1,9 Teile Champagne-Kreide/Hydroxyäthylcellulose-Gemische (1 : 1),
   1,5 Teile Natrium-dibutyl-naphthalinsulfonat,
   19,5 Teile Kieselsäure,
   19,5 Teile Champagne-Kreide,
   28,1 Teile Kaolin;

c) 25,0 Teile Wirkstoff,
   2,5 Teile Isooctylphenoxy-polyoxyäthylen-äthanol,
   1,7 Teile Champagne-Kreide/Hydroxyäthylcellulose-Gemisch (1 : 1),
   8,3 Teile Natriumaluminiumsilikat,
   16,5 Teile Kieselgur,
   46,0 Teile Kaolin;

d) 10 Teile Wirkstoff,
   3 Teile Gemisch der Natriumsalze von gesättigten Fettalkoholsulfaten,
   5 Teile Naphthalinsulfonsäure/Formaldehyd-Kondensat,
   82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu

Suspensionen jeder gewünschten Konzentration verdünnen lassen.

Emulgierbare Konzentrate

Zur Herstellung eines 10%igen emulgierbaren Konzentrates werden folgende Stoffe verwendet:

| | |
|---|---|
| 10,0 Teile | Wirkstoff, |
| 3,4 Teile | epoxydiertes Pflanzenöl, |
| 3,4 Teile | eines Kombinationsemulgators, bestehend aus Fettalkoholpolyglykoläther und Alkylaralkyl-sulfonat-Calcium-Salz, |
| 40,0 Teile | Dimethylformamid, |
| 43,2 Teile | Xylol. |

Aus einem solchen Konzentrat können durch Verdünnen mit Wasser Emulsionen jeder gewünschten Konzentration hergestellt werden.

Sprühmittel

Zur Herstellung eines 5%igen Sprühmittels werden die folgenden Bestandteile verwendet:

| | |
|---|---|
| 5 Teile | Wirkstoff, |
| 1 Teil | epoxydiertes Pflanzenöl, |
| 94 Teile | Benzin (Siedegrenzen 160—190° C). |

Beispiel 1

5,8 g 3-(3-Chlor-5-trifluormethyl-pyridyl-2-oxy)-anilin in 40 ml wasserfreiem Toluol werden mit 3,7 g 2,6-Difluorbenzoylisocyanat in 20 ml wasserfreiem Toluol versetzt. Nachdem die anfänglich exotherme Reaktion abgeklungen ist, wird das Gemisch über Nacht stehengelassen. Nach Filtration ergeben sich gelbe Kristalle, welche aus Toluol umkristallisiert werden. Man erhält auf diese Weise den N-3-(3-Chlor-5-trifluormethyl-2-pyridyloxy)-phenyl-N'-2,6-difluorbenzoylharnstoff vom Smp. 172—173° C. (Verbindung Nr. 1).

Herstellung der Ausgangsverbindung

12 g 3-Aminophenol, 7,0 g Kaliumhydroxid und 100 ml Dimethylsulfoxid werden mit 50 ml Toluol versetzt und auf ca. 150° C erwärmt. Das Gemisch wird ca. 4 Stunden stehengelassen, um das Wasser abzuscheiden. Dann wird das Toluol bei Normaldruck abdestilliert. Man kühlt auf 120° C ab und läßt bei dieser Temperatur 16,0 g 2,3-Dichlor-5-trifluormethylpyridin in 20 ml Dimethylsulfoxid zutropfen; anschließend wird 8 Stunden bei dieser Temperatur gerührt. Man läßt abkühlen und stellt das Reaktionsgemisch mit Eisessig auf pH 7 ein. Die Lösungsmittel werden dann im Vakuum vollständig abdestilliert, der Rückstand wird in Toluol aufgenommen, mehrmals mit Wasser gewaschen und über Natriumsulfat getrocknet. Das nach Verdampfen des Lösungsmittel verbleibende Öl wird mit Hexan verrieben, wobei es kristallisiert. Man erhält auf diese Weise das 3-(3-Chlor-5-trifluormethyl-pyridyl-2-oxy)-anilin vom Smp. 67—68° C.

Analog den vorstehend beschriebenen Arbeitsweisen werden die folgenden Verbindungen der Formel I hergestellt:

| Verbindung Nr. | $R_1$ | $R_2$ | Schmelzpunkt (°C) |
|---|---|---|---|
| 1 | F | F | 172—173 |
| 2 | F | H | 183—184 |
| 3 | Cl | H | 166—169,5 |
| 4 | F | Cl | |
| 5 | Cl | Cl | 183—185 |
| 6 | $CH_3$ | H | |

Beispiel 2

Wirkung gegen Musca domestica

Je 50 g frisch zubereitetes CSMA-Nährsubstrat für Maden wurden in Becher eingewogen. Von einer 1gew.-%igen acetonischen Lösung des betreffenden Wirkstoffes wurde eine bestimmte Menge auf das in den Bechern befindliche Nährsubstrat pipettiert. Nach dem Durchmischen des Substrates läßt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefäßen deponiert.

Die pro Ansatz ausgeschwemmten Puppen wurden gezählt (toxischer Einfluß des Wirkstoffes auf die Madenentwicklung). Dann wurde nach 10 Tagen die Anzahl der aus den Puppen geschlüpften Fliegen bestimmt.

Verbindungen der Formel I gemäß Beispiel 1 zeigten gute Wirkung im obigen Test.

Beispiel 3

Wirkung gegen Lucilia sericata

Zu 9 ml eines Zuchtmediums wurden bei 50° C 1 ml einer 0,5% Aktivsubstanz enthaltenden wäßrigen Lösung gegeben. Nun wurden ca. 30 frisch geschlüpfte Lucilia sericata-Larven zum Zuchtmedium gegeben, und nach 48 und 96 Stunden wurde die insektizide Wirkung durch Ermittlung der Abtötungsrate festgestellt.

Verbindungen der Formel I gemäß Beispiel 1 zeigten in diesem Test gute Wirkung gegen Lucilia sericata.

Beispiel 4

Wirkung gegen Aedes aegypti

Auf die Oberfläche von 150 ml Wasser, das sich in einem Behälter befindet, wurde so viel einer 0,1%igen actonischen Lösung des Wirkstoffes pipettiert, daß Konzentrationen von je 10, 5 und 1 ppm erhalten wurden. Nach Verdunsten des Acetons wurde der Behälter mit 30—40 2tägigen Aedes-Larven beschickt. Nach 1, 2 und 5 Tagen wurde die Mortalität geprüft.

Verbindungen der Formel I gemäß Beispiel 1 zeigten in diesem Test gute Wirkung gegen Aedes aegypti.

Beispiel 5

Insektizide Fraßgift-Wirkung

Ca. 25 cm hohe eingetopfte Baumwollpflanzen wurden mit wäßrigen Wirkstoffemulsionen besprüht, die den Wirkstoff in Konzentrationen von 100, 50, 12,5 und 0,05 ppm enthielten.

Nach dem Antrocknen des Sprühbelages wurden die Baumwollpflanzen mit Spodoptera littoralis- bzw. Heliothis virescens-Larven im dritten larvalen Stadium besiedelt. Der Versuch wurde bei 24°C und 60% relativer Luftfeuchtigkeit durchgeführt. Nach 120 Stunden wurde die %-Mortalität der Test-Insekten bestimmt.

Beispiel 6

Wirkung gegen Epilachna varivestis

Etwa 15—20 cm hohe Phaseolus vulgaris-Pflanzen (Buschbohnen) wurden mit wäßrigen, den zu prüfenden Wirkstoff in Konzentrationen von 400 ppm bzw. 800 ppm enthaltenden Emulsions-Zubereitungen besprüht. Nach dem Antrocknen des Sprühbelages wurden pro Pflanze 5 Larven von Epilachna varivestis (Mexikanischer Bohnenkäfer) im 4. larvalen Stadium angesetzt. Über die infestierten Pflanzen wurde ein Plastikzylinder gestülpt, der mit einem Kupfer-Gazedeckel abgedeckt war. Der Versuch wurde bei 28° C und 60% relativer Luftfeuchtigkeit durchgeführt.

Nach 2 und 3 Tagen wurde die akute Wirkung (%-Mortalität) bestimmt. Zur Auswertung hinsichtlich allfälligem Fraß-Schaden, (Antifeeding-Effekt), Entwicklungs- und Häutungsstörungen wurden die Versuchstiere während weiterer 3 Tage beobachtet.

Biologische Ergebnisse

Die nachstehende Tabelle zeigt Ergebnisse biologischer Prüfungen erfindungsgemäßer Verbindungen auf der Basis obiger biologischer Beispiele. Die Auswertung der Versuche erfolgte anhand der erhaltenen %-Mortalität unter Verwendung des folgenden Bewertungs-Index:

A: 80—100% Mortalität bei einer Konzentration von 0,05 ppm der geprüften Verbindung
B: 80—100% Mortalität bei einer Konzentration von 12,5 ppm der geprüften Verbindung
C: 80—100% Mortalität bei einer Konzentration von 50 ppm der geprüften Verbindung
D: 80—100% Mortalität bei einer Konzentration von 100 ppm der geprüften Verbindung
E: 80—100% Mortalität bei einer Konzentration von 400 ppm der geprüften Verbindung
F: weniger als 80% Mortalität bei einer Konzentration von 800 ppm der geprüften Verbindung

| Verbindung Nr. | pestizide Wirksamkeit | | |
|---|---|---|---|
| | Spodoptera Larven (Beispiel 5) | Heliothis Larven (Beispiel 5) | Epilachna Larven (Beispiel 6) |
| 1 | B | A | F |
| 2 | C | D | E |
| 3 | C | C | F |

**Patentansprüche**

1. Verbindung der Formel I

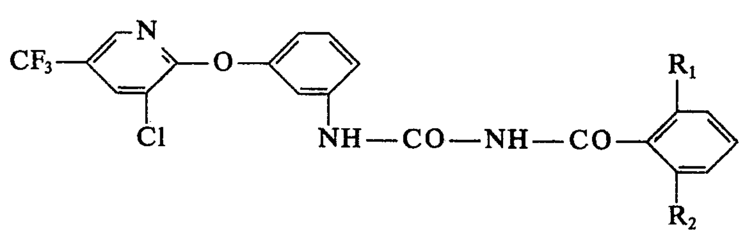

(I)

worin

$R_1$ Fluor, Chlor, Brom oder Methyl und
$R_2$ Wasserstoff, Fluor, Chlor oder Brom

bedeuten.

2. Verbindung gemäß Anspruch 1, dadurch gekennzeichnet, daß $R_1$ und $R_2$ unabhängig voneinander

Fluor oder Chlor bedeuten.

3. Verbindung gemäß Anspruch 1, dadurch gekennzeichnet, daß $R_2$ Wasserstoff bedeutet.

4. Verbindung gemäß Anspruch 2 der Formel

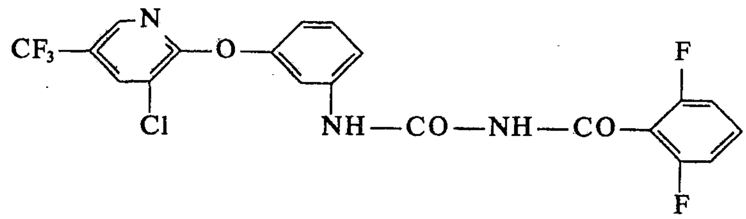

5. Verbindung gemäß Anspruch 2 der Formel

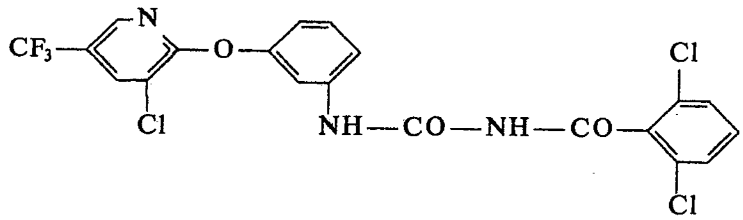

6. Verbindung gemäß Anspruch 3 der Formel

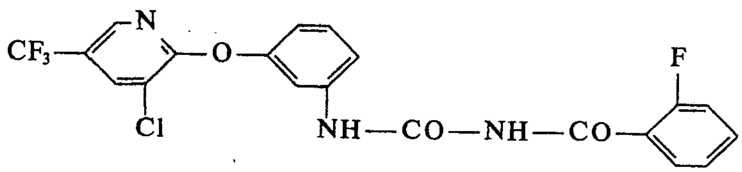

7. Verbindung gemäß Anspruch 3 der Formel

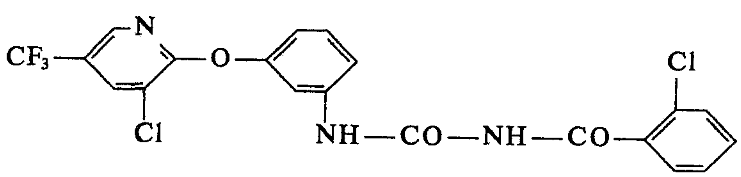

8. Verbindung der Formel II

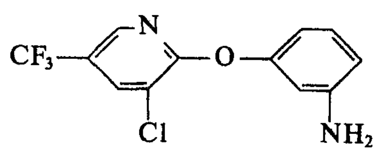

(II)

9. Verbindung der Formel IV

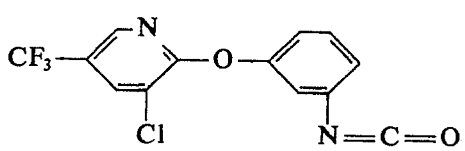

(IV)

10. Verfahren zur Herstellung einer Verbindung gemäß den Ansprüchen 1 bis 7, dadurch gekennzeichnet, daß man

a) die Verbindung der Formel II

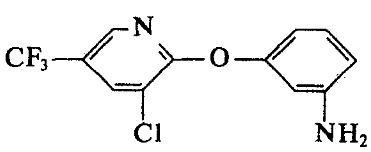

(II)

mit einer Verbindung der Formel III

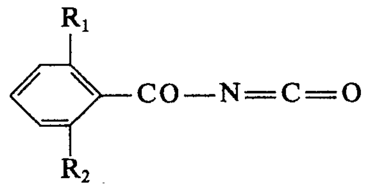

(III)

oder

b) die Verbindung der Formel IV

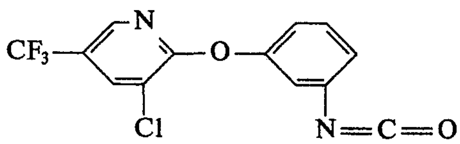

(IV)

mit einer Verbindung der Formel V

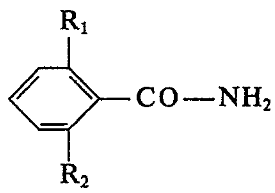

(V)

oder

c) die Verbindung der Formel II mit einer Verbindung der Formel VI

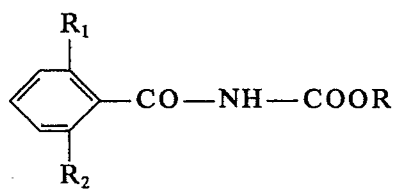

(VI)

umsetzt, wobei in den Formeln III, V und VI die Reste $R_1$ und $R_2$ die in den Ansprüchen 1 und 3 angegebenen Bedeutungen haben und R für einen $C_1-C_8$-Alkylrest, der gegebenenfalls mit Halogen substituiert ist, steht.

11. Schädlingsbekämpfungsmittel, welches als aktive Komponente eine Verbindung gemäß den Ansprüchen 1 bis 7 zusammen mit geeigneten Trägern und/oder anderen Zuschlagstoffen enthält.

12. Verwendung einer Verbindung gemäß den Ansprüchen 1 bis 7 zur Bekämpfung von Schädlingen, vorzugsweise von Insekten.

13. Verwendung gemäß Anspruch 12 zur Bekämpfung larvaler Stadien pflanzenschädigender Insekten.

## Claims

1. Compound of the formula I

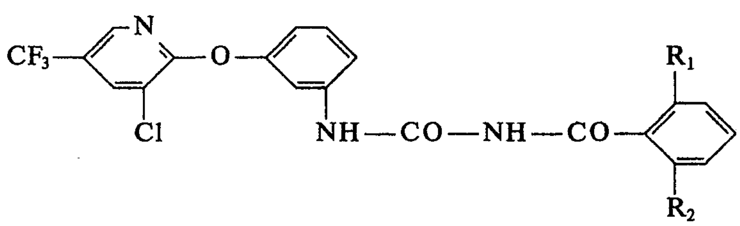

(I)

wherein

$R_1$ is fluorine, chlorine, bromine or methyl, and
$R_2$ is hydrogen, fluorine, chlorine or bromine.

2. Compound according to Claim 1, characterised in that $R_1$ and $R_2$ independently of one another are fluorine or chlorine.

3. Compound according to Claim 1, characterised in that $R_2$ is hydrogen.

4. Compound according to Claim 2 of the formula

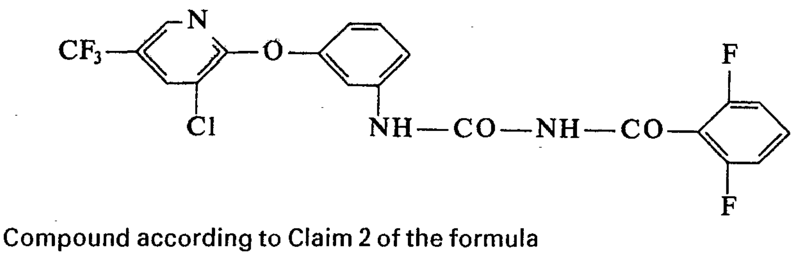

5. Compound according to Claim 2 of the formula

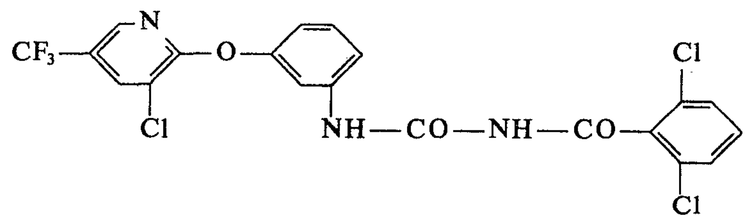

6. Compound according to Claim 3 of the formula

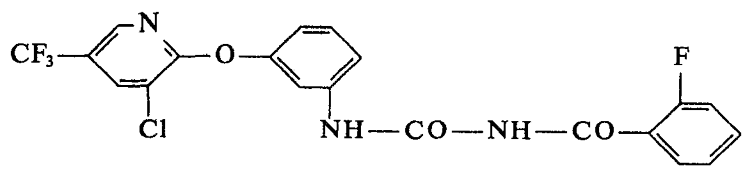

7. Compound according to Claim 3 of the formula

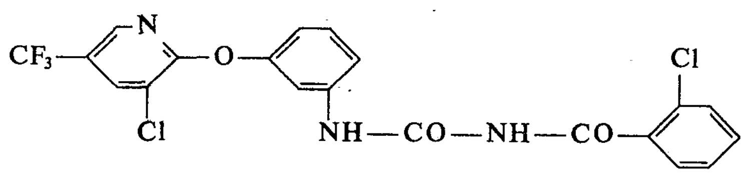

8. Compound of the formula II

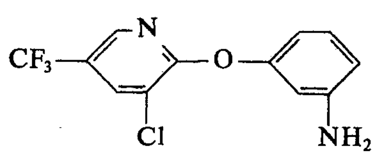

(II)

9. Compound of the formula IV

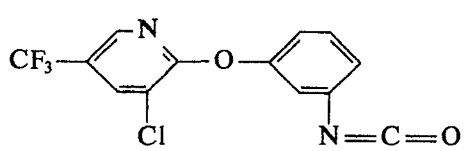

(IV)

10. Process for producing a compound according to any one of Claims 1 to 7, characterised in that

a) the compound of the formula II

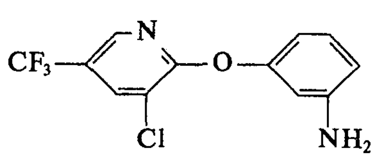

(II)

is reacted with a compound of the formula III

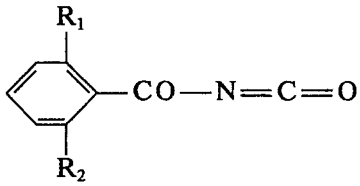

(III)

or

b) the compound of the formula IV

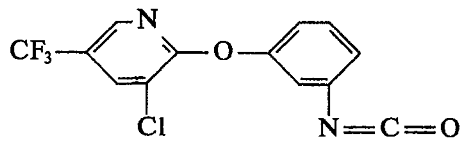

(IV)

is reacted with a compound of the formula V

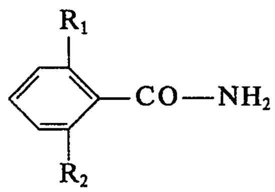

(V)

or

c) the compound of the formula II is reacted with a compound of the formula VI

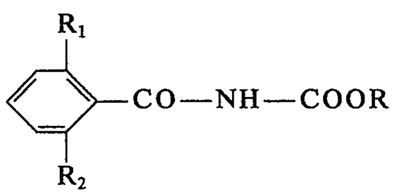

(VI)

the symbols $R_1$ and $R_2$ in the formulae III, V and VI having the meanings defined in Claims 1 to 3, and R being a $C_1-C_8$-alkyl group which is unsubstituted or substituted by halogen.

11. Pesticidal composition which contains as active ingredient a compound according to any one of Claims 1 to 7, together with suitable carriers and/or other additives.

12. Use of a compound according to Claims 1 to 7 for combating pests, particularly insects.

13. Use according to Claim 12 for combating larval stages of insects which damage plants.

**Revendications**

1. Composé de formule I

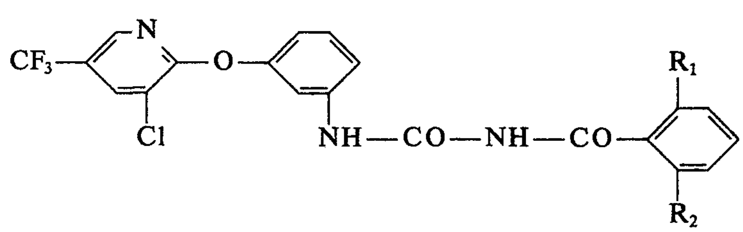

(I)

dans laquelle $R_1$ représente le fluor, le chlore, le brome ou un groupe méthyle, et $R_2$ représente l'hydrogène, le fluor, le chlore ou le brome.

2. Composé selon la revendication 1, caractérisé en ce que $R_1$ et $R_2$ représentent chacun indépendamment l'un de l'autre, le fluor ou le chlore.

3. Composé selon la revendication 1, caractérisé en ce que $R_2$ représente l'hydrogène.

4. Composé selon la revendication 2, de formule

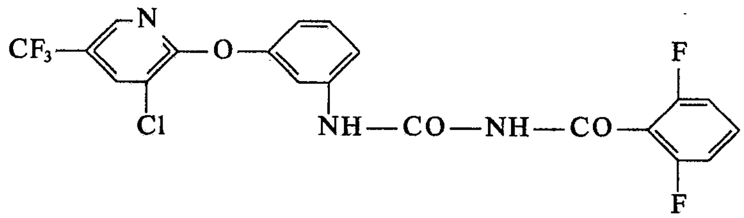

5. Composé selon la revendication 2, de formule

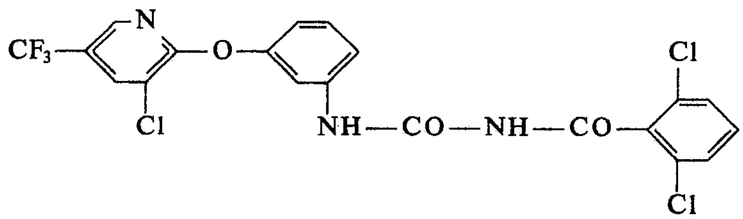

6. Composé selon la revendication 3, de formule

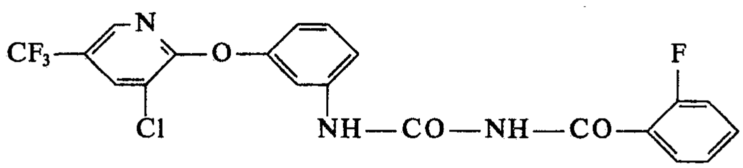

7. Composé selon la revendication 3, de formule

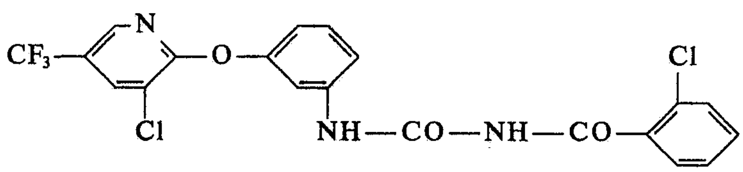

8. Composé de formule II

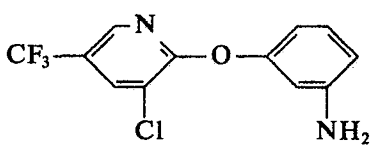

(II)

9. Composé de formule IV

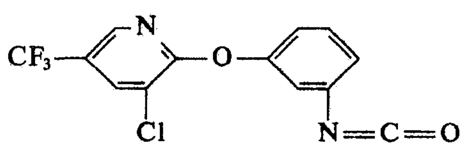

(IV)

10. Procédé de préparation d'un composé selon les revendications 1 à 7, caractérisé en ce que;

a) on fait réagir le composé de formule II

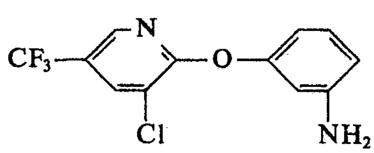

(II)

avec un composé de formule III

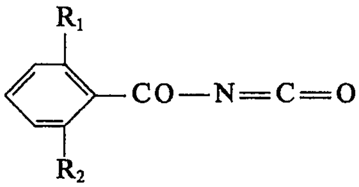

(III)

ou bien

b) on fait réagir le composé de formule IV

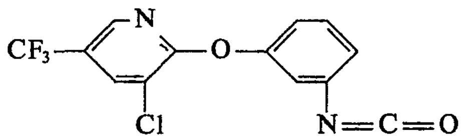

(IV)

avec un composé de formule V

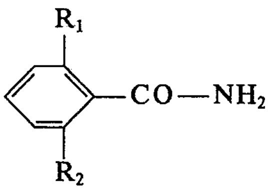

(V)

ou bien

c) on fait réagir le composé de formule II avec un composé de formule VI

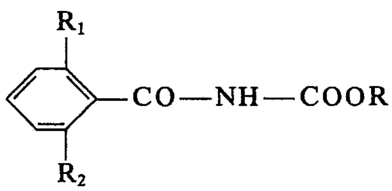

(VI)

les symboles $R_1$ et $R_2$ ayant dans les formules III, V et VI les significations indiquées dans les revendications 1 à 3 et R représentant un groupe alkyle en $C_1-C_8$ éventuellement substitué par des halogènes.

11. Produit pesticide contenant en tant que composant actif un composé selon les revendications 1 à 7 avec des véhicules et/ou d'autres additifs appropriés.

12. Utilisation d'un composé selon les revendications 1 à 7, pour la lutte contre les parasites, de préférence les insectes.

13. Utilisation selon la revendication 12, pour la lutte contre les stades larvaires des insectes nuisibles pour les végétaux.